Europäisches Patentamt

European Patent Office

Office européen des brevets

(11) Veröffentlichungsnummer: **0 460 534 A2**

# EUROPÄISCHE PATENTANMELDUNG

(21) Anmeldenummer: 91108854.0

(22) Anmeldetag: 29.05.91

(51) Int. Cl.⁵: **A61L 27/00**

(30) Priorität: 29.05.90 DE 4017284
26.07.90 DE 4023786
26.07.90 DE 4023787

(43) Veröffentlichungstag der Anmeldung:
**11.12.91 Patentblatt 91/50**

(84) Benannte Vertragsstaaten:
**DE FR IT**

(71) Anmelder: **Schumann, Prof.Dr.med. K.**
**Franz-Wiedemeier-Strasse 27**
**W-7900 Ulm(DE)**

(72) Erfinder: **Schumann, Prof.Dr.med. K.**
**Franz-Wiedemeier-Strasse 27**
**W-7900 Ulm(DE)**

(74) Vertreter: **Zinnecker, Armin, Dipl.-Ing.**
**Lorenz-Seidler-Gossel et al**
**Widenmayerstrasse 23**
**W-8000 München 22(DE)**

(54) **Masse, enthaltend Kohlefasermaterial und 1 oder mehr Glasionomer-Material(ien) sowie daraus hergestellte Prothese und Verfahren und Vorrichtung zu deren Herstellung.**

(57) Gegenstand der Erfindung ist eine erhärtbare Masse enthaltend Kohlefasermaterial und 1 oder mehr Glasionomer-Material(ien) zur Verwendung in der Knochenchirurgie.

Diese Prothese vermag dauerhafte Verbindungen zu bilden und Distanzen zu überbrücken und hat hygroskopische Eigenschaften und kann dadurch Flüssigkeiten absorbieren.

EP 0 460 534 A2

Die Erfindung betrifft eine erhärtbare Masse zur Verwendung in der Knochenchirurgie sowie eine daraus hergestellte Prothese und ein Verfahren und eine Vorrichtung zu deren Herstellung.

Bei Knochenzerstörungen bzw. -defekten des menschlichen und tierischen Körpers werden Prothesen benötigt.

Bei vielen Erkrankungen kommt es zu Zerstörungen des Knochens. Unfälle, Entzündungen und Tumore sind die häufigste Ursache. Beispielsweise in solchen Fällen sind Prothesen erforderlich.

Der Erfindung liegt die Aufgabe zugrunde, eine erhärtbare Masse zur Verwendung in der Knochenchirurgie, die besonders gut verwendbar ist und dabei insbesondere dauerhafte Verbindungen zu bilden und Distanzen zu überbrücken vermag sowie hygroskopische Eigenschaften hat und dadurch Flüssigkeiten absorbieren kann, und eine daraus hergestellte dauerhafte Prothese zu schaffen.

Dies wurde überraschenderweise durch die erfindungsgemäße erhärtbare bzw. abbindbare Masse zur Verwendung in der Knochenchirurgie, welche Kohlefasermaterial und 1 oder mehr Glasionomer-Material(ien) enthält, erreicht.

Nach einer zweckmäßigen Ausführungsform ist die erfindungsgemäße Masse ein Osteosynthesematerial.

Vorzugsweise liegen die Kohlefasern in einem Kohlefaserbündel vor. Ein solches Kohlefasermaterial wird beispielsweise von der Firma Braun (Melsungen) als Bandmaterial vertrieben. Die Kohlefasern können auch in Geflechtform bzw. Mattenform oder auch als Einzelfasern vorliegen.

Zweckmäßig sind die Kohlefasern mit dem bzw. den Glasionomer-Material(ien) getränkt.

Nach einer bevorzugten Ausführungsform sind die Kohlefasern mit einem Harz vorgetränkt. Besonders bevorzugt ist dieses Harz ein Epoxyharz. Diese Ausführungsform ist von besonderer Bedeutung bei der Verwendung in Bereichen, in welchen nach der Osteosynthese zur Protheseherstellung Scherkräfte zu erwarten sind, wie bei Knochenfrakturen des Bewegungsapparates und Unterkiefers.

Es ist auch bevorzugt, daß das/die Glasionomer-Material(ien) aus pulverförmigem Calciumaluminiumsilikatglas und/oder Calciumaluminiumfluorsilikatglas, gegebenenfalls mit einem Gehalt an Natrium und/oder Phosphor, aus einem Polymer und/oder Copolymer einer ungesättigten Carbonsäure und aus einem Chelatbildner als Abbindestarter hergestellt ist/sind.

Eine nähere Beschreibung des Glasionomer-Materiales und seiner Eigenschaften ist in der DOS 39 30 921 enthalten.

Vorzugsweise beträgt das Gewichtsverhältnis des Kohlefasermateriales zu dem/den Glasionomer-Material(ien) 1 : 4 bis 1 : 8, insbesondere 1 : 5 bis 1 : 7.

Gegenstand der Erfindung ist auch eine Prothese, welche aus einer erfindungsgemäßen Masse hergestellt ist.

Ferner ist Gegenstand der Erfindung ein Verfahren zur Herstellung der erfindungsgemäßen Prothese, welches dadurch gekennzeichnet ist, daß zur Herstellung einer festen Knochenverbindung das Kohlefasermaterial mit, zweckmäßig kurz, vorzugsweise nicht mehr als 3 Minuten, vor der Anwendung angemischtem/angemischten Glasionomer-Material(ien), zweckmäßig mit leimiger Konsistenz, getränkt und in Knochendefekte eingeführt wird und das/die Glasionomer-Material(ien) zu [einem] Glasionomer-Zement(en) erhärten gelassen wird/werden. Zweckmäßig wird der etwaig verwendete Abbindestarter kurz vor der Anwendung dem/den Glasionen-Material(ien) zugesetzt. Vorteilhaft werden die Kohlefasern in einem Kohlefaserbündel eingesetzt, wobei sie wahlweise als Ganzes genutzt oder als Geflecht verlegt werden.

Die Erfindung kann in den folgenden Bereichen besonders vorteilhaft angewandt werden:

a) Zur Behebung von Knochendefekten nach Unfällen, Entzündungen, Tumoren bzw. nach Operationen, die nach Unfällen, Entzündungen, Tumoren durchgeführt werden müssen. Insbesondere können mit der erfindungsgemäßen Prothese auch Distanzen überbrückt werden, die auf Grund von Unfällen oder sonst in Knochen entstehen, beispielsweise nach operativer Beseitigung von Splitterstücken aus Knochen, insbesondere Schädelbasisknochen.

b) Zur Schaffung dauerhafter Verbindungen bei Knochenbrüchen. Auch hier ist besonders vorteilhaft, daß Distanzen überbrückt werden können.

c) Als Mittelohrprothesen und deren Fixierung. Hierbei können ebenfalls Distanzen durch Kohlefasern überbrückt werden.

Die Kohlefasern der erfindungsgemäßen Massen bzw. Prothesen schaffen ein besonders flexibles und formbares sowie den jeweiligen Gegebenheiten besonders gut anpaßbares System für den Anwender. Das Kohlefasermaterial kann nach dem jeweiligen Verwendungszweck gewählt werden. Je nach Anwendungsfall ist die Verwendung von Kohlefaserbündeln oder von Kohlefasergeflechten oder Einzelfasern vorteilhaft. Das Kohlefasermaterial kann vor der Anwendung zugeschnitten und auch entsprechend dem Anwendungszweck in weiten Grenzen frei verformt worden sein.

Die Anwendung des Kohlefasermaterials hat den weiteren Vorteil, daß es hygroskopische Eigenschaften hat und damit auch in den Fällen die Abbindung des feuchtigkeitsempfindlichen Glasionomer-Zementes ermöglicht, in denen viel Flüssigkeit anfällt. Ein derartiges "Zuviel" von Flüssigkeit im Operationsfeld tritt beispielsweise bei der Versorgung von Knochendefekten im Bereich der Schädelbasis und gleichzeitig bestehendem Liquorfluß auf. Bei Röhrenknochen tritt Blut aus den eröffneten Markräumen aus. In diesen Fällen kann die überschüssige Flüssigkeit durch die Kohlefasern aufgesaugt und aufgenommen werden. Damit können Voraussetzungen geschaffen werden, die zur optimalen Aushärtung des Glasionomer-Zementes notwendig sind. Hierfür wird zwar etwas, aber nicht zu viel Feuchtigkeit benötigt.

Weiterhin ist Gegenstand der Erfindung eine Vorrichtung zur Durchführung des erfindungsgemäßen Verfahrens, welches dadurch gekennzeichnet ist, daß sie ein Hilfsinstrument, insbesondere ein Kalfateisen, eine Knopfsonde und/oder eine Haltezange, wie Repositionszange und/oder eine Kugelnadel ist.

Kalfateisen ist besonders vorteilhaft zur Schaffung dauerhafter Verbindungen in feuchten Bereichen, z. B. in der Schädelbasischirurgie und bei eröffneten Markräumen. Sogenannte "Kalfateisen" sind in vergrößerter Form bereits aus dem Schiffsbau bekannt. Die Kohlefasern funktionieren in diesem Sinne als "Kalfatwolle". Der Vorteil der "Kalfattechnik" besteht darin, daß das Zuviel von Flüssigkeiten unterbunden wird.

Zur Schaffung dauerhafter Verbindungen [vgl. oben b)] in Arealen mit Scherkräften ist es vorteilhaft, Halteeinrichtungen, beispielsweise Halte- bzw. Repositionszangen in verschiedenen Größen, die beispielsweise von der Herstellerfirma Synthes vertrieben werden, zu verwenden. Auch der Einsatz von Tuchklammern ist möglich.

Für die Schaffung und Fixierung von Mittelohrprothesen [vgl. oben c)] werden zweckmäßig Kugelnadeln zusätzlich verwendet. Diese Nadeln weisen im Bereich der "Spitze" eine Kugel von beispielsweise 2 mm Durchmesser auf.

**Patentansprüche**

1. Erhärtbare Masse, enthaltend Kohlefasermaterial und 1 oder mehr Glasionomer-Material(ien) zur Verwendung in der Knochenchirurgie.

2. Masse nach Anspruch 1, dadurch gekennzeichnet, daß die Kohlefasern in 1 oder mehr Kohlefaserbündel(n) vorliegen.

3. Masse nach Anspruch 1 oder 2, dadurch gekennzeichnet, daß die Kohlefasern mit dem bzw. den Glasionomer-Material(ien) getränkt sind.

4. Masse nach einem der Ansprüche 1 bis 3, dadurch gekennzeichnet, daß die Kohlefasern mit einem Harz vorgetränkt sind.

5. Masse nach einem der Ansprüche 1 bis 4, dadurch gekennzeichnet, daß das/die Glasionomer-Material(ien) aus pulverförmigem Calciumaluminiumsilikatglas und/oder Calciumaluminiumfluorsilikatglas, gegebenenfalls mit einem Gehalt an Natrium und/oder Phosphor, aus einem Polymer und/oder Copolymer einer ungesättigten Carbonsäure und aus einem Chelatbildner als Abbindestarter besteht/bestehen.

6. Masse nach einem der Ansprüche 1 bis 5, dadurch gekennzeichnet, daß das Gewichtsverhältnis des Kohlefasermateriales zu dem/den Glasionomer-Material(ien) 1 : 4 bis 1 : 8 beträgt.

7. Masse nach einem der Ansprüche 1 bis 6, dadurch gekennzeichnet, daß das Harz, mit welchem die Kohlefasern vorgetränkt sind, ein Epoxyharz ist.

8. Masse nach Anspruch 1 bis 7, dadurch gekennzeichnet, daß sie ein Osteosynthesematerial ist.

9. Prothese, dadurch gekennzeichnet, daß sie aus einer Masse nach Anspruch 1 bis 8 hergestellt ist.

10. Verfahren zur Herstellung der Prothese nach Anspruch 9, dadurch gekennzeichnet, daß man zur Herstellung einer festen Knochenverbindung das Kohlefasermaterial mit, zweckmäßig kurz, vorzugsweise nicht mehr als 3 Minuten, vor der Anwendung angemischtem/angemischten Glasionomer-Material(ien) tränkt und das/die Glasionomer-Material(ien) zu [einem] Glasionomer-Zement(en) erhärten läßt.

11. Vorrichtung zur Durchführung des Verfahrens nach Anspruch 10, dadurch gekennzeichnet, daß sie ein Hilfsinstrument, insbesondere ein Kalfateisen, eine Knopfsonde und/oder eine Haltezange und/oder eine Kugelnadel ist.